Europäisches Patentamt

European Patent Office

Office européen des brevets

(11) Veröffentlichungsnummer: **0 415 152 A1**

# (12) EUROPÄISCHE PATENTANMELDUNG

(21) Anmeldenummer: **90115366.8**

(22) Anmeldetag: **10.08.90**

(51) Int. Cl.⁵: **A61B 1/04**, H04N 5/235

(30) Priorität: **25.08.89 DE 3928052**

(43) Veröffentlichungstag der Anmeldung:
**06.03.91 Patentblatt 91/10**

(84) Benannte Vertragsstaaten:
**DE FR GB NL**

(71) Anmelder: **Richard Wolf GmbH**
**Pforzheimer Strasse 32**
**W-7134 Knittlingen(DE)**

(72) Erfinder: **Ams, Felix**
**Gründle Strasse 5**
**W-7539 Kämpfelbach(DE)**
Erfinder: **Schäfer, Roland**
**Lugenbergstrasse 9**
**W-7518 Bretten-Dürrenbüchig(DE)**

(74) Vertreter: **Vollmann, Heiko, Dipl.-Ing. et al**
**Patentanwälte Wilcken & Wilcken,**
**Musterbahn 1**
**W-2400 Lübeck 1(DE)**

(54) **Automatische Lichtregelung für ein Endoskop.**

(57) In einer Einrichtung zum automatischen Regeln der für das Beleuchtungslicht eines mit einer Videokamera (5) ausgerüsteten Endoskopes (1) einsetzbaren Lichtquelle (2) ist eine Signalverarbeitungseinrichtung (3) mit einer Fenstererzeugungseinrichtung (14) sowie mit einer das Fenster visuell darstellenden und optisch abgrenzenden Fenstereinblendeinrichtung (23) vorgesehen. In dem auf dem Monitor (7) dargestellten Videobild wird ein Meßfenster (18) erzeugt und als Rahmen eingeblendet. Das für die Regelung der Lichtmenge eingesetzte Videosignal wird nur innerhalb des Meßfensters (18) integriert .

EP 0 415 152 A1

## AUTOMATISCHE LICHTREGELUNG FÜR EIN ENDOSKOP

Die Erfindung geht aus von einer Einrichtung zum automatischen Regeln der für das Beleuchtungslicht eines Endoskopes vorgesehenen Lichtquelle, mit einem das Beleuchtungslicht übertragenden, an das zu betrachtende Objekt heranzuführenden Lichtleiter, einem mit einer Videokamera in Verbindung stehenden Bildleiter und einer Signalverarbeitungsschaltung für das von der Videokamera erzeugte Signal.

Zur Betrachtung schwer zugänglicher Stellen, z.B. Körperhöhlen, wird häufig ein Endoskop eingesetzt. Zur Auswertung und Dokumentation des Betrachteten besteht die Möglichkeit, durch eine extrakorporal am Endoskop angebrachte Kamera Aufnahmen von den betrachteten Körperstellen zu machen. Bei Verwendung von Fotokameras können fotografische Aufnahmen gemacht werden und bei Einsatz eines Lichtwandlerelementes, beispielsweise eines CCD-Elementes, das die optischen Signale in elektrische Signale (Videosignale) umwandelt, können Aufzeichnungen mit einem Videorecorder hergestellt werden. Um bei solchen Aufnahmen gute Ergebnisse erzielen zu können, d.h. Bilder erzeugen zu können, die beispielsweise eine exakte Diagnose ermöglichen, muß dafür gesorgt werden, daß das interessierende Objekt optimal ausgeleuchtet wird. Wird von einer Lichtquelle eine konstante Lichtmenge geliefert, so kann es vorkommen, daß die betrachteten Körperstellen zum einen sehr stark reflektieren, d.h. überstrahlt oder zum anderen zu schwach beleuchtet werden. Folglich ist für die Lichtquelle eine automatische Lichtregelung erforderlich, die bei variablen Objektabständen die optimale Lichtmenge ermittelt und einstellt.

Regelungsschaltungen für die Regelung der Beleuchtungsstärke von Lichtquellen sind bekannt. So ist beispielsweise in der DE-PS 35 09 825 ein Lichtprojektor mit regelbarer Beleuchtungsstärke beschrieben, bei dem es möglich ist, die Ausleuchtung eines Objektes in Abhängigkeit seines Abstandes vom distalen Ende des Endoskopes richtig einzustellen und auf einen Maximalwert zu begrenzen. Dazu wird ein Soll-Ist-Wertvergleich durchgeführt und als Sollwert die maximal zulässige Amplitude des Videosignals herangezogen. Durch diese Spitzenwertregelung wird erreicht, daß die hellsten Bildteile nicht überstrahlen. Nachteilig bei dieser Lösung ist jedoch, daß der Hintergrund des Bildes in der Regel zu dunkel wird.

Aus der DE-PS 31 18 341 ist weiter eine Lichtregelung für ein Endoskop bekannt, bei dem die Bildaufnahme mit einer Fernsehkamera erfolgt. Hier wird die Lichtmenge, die auf den Lichtleiter trifft, so geregelt, daß unabhängig von Änderungen des Abstandes zwischen Beobachtungsobjekt und distalem Endoskopende die Spannung des Videosignals auf im wesentlichen konstantem Pegel (Spannungspegel) gehalten wird. Da bei dieser Form der Lichtregelung die Bildhelligkeit mit Hilfe des Mittelwertes des Videosignales geregelt wird, besteht der Nachteil dieser Lichtregelung darin, daß das interessierende Objekt nicht immer richtig beleuchtet wird.

Schließlich ist aus der DE-OS 37 43 090 eine Beleuchtungsregelung für ein Endoskop bekannt, bei der das Beleuchtungslicht und die Helligkeit des abgebildeten Gegenstandes automatisch unabhängig von der durch den Bildleiter übertragbaren Bildgröße geregelt wird. Dabei wird der infolge der Verwendung von Endoskopen mit unterschiedlichen Bildkreisdurchmessern und damit unterschiedlichen Bildbereichen veränderte Pegel des Videosignals dadurch berücksichtigt, daß die Bildbreite des endoskopischen Bildes Zeile für Zeile abgetastet und die größte Breite durch eine Spitzenwerthalteschaltung als Spannungspegel gespeichert und zum eigentlichen Istwert des Videosignals addiert wird.

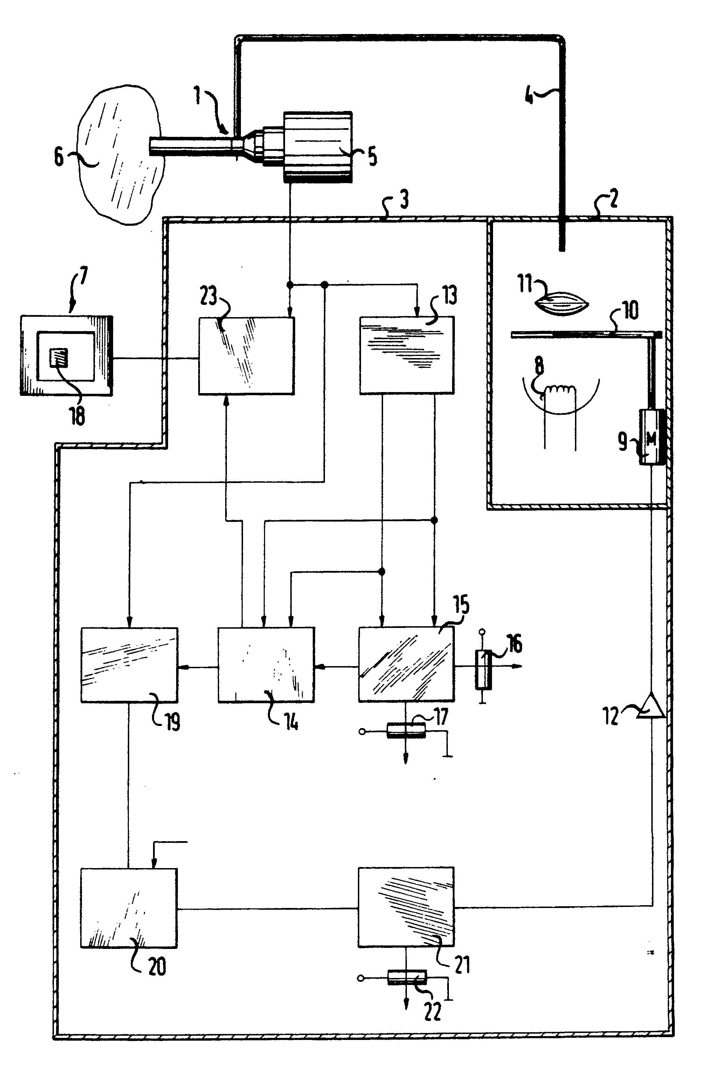

Eine weitere Einrichtung zur Lichtregelung für ein Endoskop ist der DE-OS 38 18 125 zu entnehmen. Bei dieser Einrichtung wird die Änderung des Bildkreisdurchmessers bei Verwendung von Endoskopen mit unterschiedlichen Durchmessern berücksichtigt und die Lichtmenge entsprechend geregelt. Dazu werden die Signalwerte von Pixeln einer Festkörper-Bildaufnahmeeinrichtung darauf überprüft, ob sie einen festgelegten Dunkelwert überschreiten. Aus dem Ergebnis dieser Prüfung wird die der Querschnittsfläche des Bildleiters entsprechende Bildabtastfläche festgestellt und durch Auswählen eines Korrektursignals ein Lichtregelsignal erzeugt, durch das die dem Lichtleiter zugeführte Lichtmenge geregelt wird. Diese Lichtregeleinrichtung hat den Nachteil, daß die in der Zeichnung der OS gestrichelt gezeichnete Quadratfläche und nicht die eigentliche Bildkreisfläche des Endoskopes zur Regelung des Videosignals benutzt wird.

Schließlich sei noch auf die US-PS 4 628 362 hingewiesen, die eine "Nachlaufsteuerung" eines Analog-Digital-Wandlers zur optimalen Nutzung des Eingangsspannungsbereiches des Videosignals beschreibt. Hierzu wird im Videobildbereich ein aktives Fenster definiert, aus dem ein Signal abgeleitet wird, mit dem der volle Dynamikbereich des Analog-Digital-Wandlers genutzt wird. Diese Erfindung betrifft keine Lichtmengensteuerung mit Flächenerfassung.

Es ist daher die Aufgabe der Erfindung, eine automatische Regelung für das Beleuchtungslicht eines Endoskopes zu schaffen, die eine für Videoaufnahmen optimale Ausleuchtung des Beobachtungsobjektes sowohl bei variablen Objektabständen, also auch unabhängig von dem jeweiligen Bildkreisdurchmesser des Endoskops, sicherstellt.

Diese Aufgabe wird erfindungsgemäß dadurch gelöst, daß die Signalverarbeitungsschaltung mit einer schaltbaren Fenstererzeugungsschaltung ausgestattet ist, die in das durch die Videokamera aufgenommene Videobild ein Meßfenster einbeschreibt und einen Integrator so steuert, daß das zur Regelung der Lichtmenge eingesetzte Videosignal nur innerhalb des Meßfensters integriert wird. Dabei kann auch eine manuell betätigbare Positionssteuerung zur Positionierung des Meßfensters innerhalb des Videobildes sowie zur Veränderung der Fenstergröße vorgesehen sein.

Damit der Anwender erkennen kann, welcher Teil des Bildes zur Helligkeitssteuerung herangezogen wird, kann eine Fenstereinblendeinrichtung vorgesehen sein, die das Einblenden des Meßfensters in das Videobild als Rahmen erlaubt.

Die Erfindung ist nachstehend anhand eines in der Zeichnung dargestellten Ausführungsbeispieles näher erläutert.

Die Gesamtordnung der erfindungsgemäßen Einrichtung umfaßt im wesentlichen ein Endoskop 1, eine dasselbe mit Beleuchtungslicht versorgende Lichtquelleneinrichtung 2 und eine Signalverarbeitungseinrichtung 3. Zur Leitung des Beleuchtungslichtes an das distale Ende des Endoskopes 1 ist ein dieses mit der Lichtquelleneinrichtung 2 verbindendes Lichtleitkabel 4 vorgesehen. Das Endoskop 1 ist mit einer Videokamera 5 ausgerüstet, die das von dem Endoskop 1 weitergeleitete Bild, beispielsweise aus einer Körperhöhle 6, aufnimmt, in ein Videosignal umwandelt und der Signalverarbeitungseinrichtung 3 sowie einem Monitor 7 zuführt. Auf diesem wird das von der Signalverarbeitungseinrichtung 3 ausgegebene Videosignal in Form eines Bildes des betrachteten Gegenstandes dargestellt.

Die Lichtquelleneinrichtung 2 umfaßt eine Lampe 8, einen Motor 9, eine Blende 10 und eine Einrichtung 11 zur Fokussierung und Filterung des von der Lampe abgegebenen Lichtes, das dann dem Lichtleiter 4 zugeführt wird. Der Motor 9 der Lichtquelleneinrichtung 2 dient der Betätigung der Blende 10, die je nach benötigter Lichtmenge zu öffnen oder zu schließen ist. Die hierzu benötigten Steuersignale werden in der Signalverarbeitungseinrichtung 3 erzeugt und über einen Motortreiber 12 zur Steuerung von Drehzahl und Drehrichtung des Motors an diesen ausgegeben.

In der Signalverarbeitungseinrichtung 3 werden durch eine Synchronabtrennstufe 13 das Horizontal- und Vertikalsynchronsignal aus dem ankommenden Videosignal herausgefiltert und einer Fenstererzeugungseinrichtung 14 und einer Positionssteuerung 15 zugeführt. Mit diesen Synchronsignalen wird in der Positionssteuerung 15 ein die Bildzeilen zählender Zeilenzähler angesteuert. Der Zählerstand eines von einem Oszillator getakteten Vergleichszählers kann mit dem Regler 16 erhöht oder erniedrigt werden, wodurch die vertikale Position eines Meßfensters 18 festgelegt wird. Bei Gleichheit der Werte von Zeilenzähler und Vergleichszähler wird durch einen Komparator ein Zeitglied mit einstellbarer Zeit in der Fenstererzeugungseinrichtung 14 getriggert. Die eingestellte Zeit des Zeitglie des entspricht dabei der Höhe des Meßfensters 18.

Mit dem Horizontal-Synchronsignal wird ein von einem Bildpunktoszillator getakteter Bildpunktzähler angesteuert. Über einen zweiten, vom Bildpunktoszillator getakteten Vergleichszähler für die Bildpunkte kann der Zählerstand mit Hilfe des Reglers 17 erhöht oder erniedrigt werden, wodurch die horizontale Position des Meßfensters 18 festgelegt wird. Bei Gleichheit der Werte von Bildpunktzähler und Bildpunktvergleichszähler wird durch einen zweiten Komparator ein zweites Zeitglied mit einstellbarer Zeit in der Fenstererzeugungseinrichtung 14 getriggert. Die an diesem Zeitglied eingestellte Zeit entspricht der Breite des Meßfensters 18.

In der Fenstererzeugungseinrichtung 14 werden die Zeitgliedausgänge durch ein Und-Gatter verknüpft, einem Integrator 19 zugeführt und dort durch ein zweites Und-Gatter mit einer Meßfenster-Wählschaltung verknüpft. Wird die Lichtregelung mit Fenstermessung gewählt, so inte griert der Integrator 19 mit steuerbarer Integrationszeit und Zeitkonstante das Videosignal nur innerhalb des Bereiches des Meßfensters 18. Am Ende eines Halbbildes wird der Integrationsendwert in einer Abtast-Halteschaltung 20 gespeichert und als Istwert dem Soll-Ist-Wert-Vergleicher 21 zugeführt, und anschließend der Integrator 19 gelöscht. Das Ergebnis des Vergleiches zwischen dem Istwert und dem vom Anwender durch einen Regler 22 vorgewählten Sollwert wird dem Motortreiber 12, der den Motor 9 der Lichtquelleneinrichtung 2 steuert, zugeführt. Die Signale der Zeitgliedausgänge der Fenstererzeugungseinrichtung 14 werden noch auf eine Fenstereinblendeinrichtung 23 geführt, die in das Videobild einen Rahmen einblendet, wodurch der Betrachter erkennen kann, welcher Teil des Bildes für die Helligkeitssteuerung gewählt ist.

Bei der erfindungsgemäßen automatischen Regeleinrichtung für das Beleuchtungslicht von Endoskopen kann zwischen Mittelwertregelung über den gesamten Bildbereich oder den variablen Fensterbereich gewählt werden. Dabei beginnt die Beleuchtungsregelung mit Fenstermessung erst, wenn die Auswahl des Meßfensters 18 getroffen ist, wodurch vermieden wird, daß beim Verändern des Fensterbereiches ständig Beleuchtungsschwankungen auftreten.

Um beispielsweise bei Störungen der Regelung mit der Lichtquelleneinrichtung 2 arbeiten zu können, ist bei der erfindungsgemäßen Einrichtung eine zusätzliche Einstellvorrichtung zur manuellen Regelung der abgegebenen Lichtmenge vorgesehen. Eine weitere zusätzliche Vorrichtung schaltet bei fehlendem oder fehlerhaftem Videosignal automatisch auf manuellen Betrieb um, und es wird insbesondere in diesem Falle die Blende 10 vollständig geschlossen.Dadurch ist vermeidbar, daß beispielsweise bei nicht angeschlossener Videokamera 5 der Anwender geblendet wird oder daß am Patienten eine hohe Wärmebelastung auftritt, wenn infolge des fehlenden Videosignales die Regelung die Blende 10 der Lichtquelleneinrichtung 2 vollständig öffnen würde.

In weiterer Ausbildung der Erfindung ist für die Positionierung des Meßfensters 18 z.B. auch der Einsatz eines Joysticks oder ähnliches möglich.

Ebenso ist für die Helligkeitsregelung nicht nur die Mittelwertmessung, sondern auch die Maximalwertmessung oder die mittenbetonte Mittelwertmessung möglich.

## Ansprüche

1. Einrichtung zum automatischen Regeln der für das Beleuchtungslicht eines Endoskopes vorgesehenen Lichtquelle mit einem das Beleuchtungslicht übertragenden, an das zu betrachtende Objekt heranzuführenden Lichtleiter und einem mit einer Videokamera verbundenen Bildleiter sowie einer Signalverarbeitungseinrichtung für das von der Videokamera erzeugte Signal, dadurch gekennzeichnet, daß die Signalverarbeitungseinrichtung (3) eine Fenstererzeugungseinrichtung (14) sowie eine das Fenster visuell darstellende und optisch abgrenzende Fenstereinblendeinrichtung (23) aufweist, durch die in dem auf dem Monitor dargestellten, durch die Videokamera (5) aufgenommenen Videobild ein Meßfenster (18) erzeugt und als Rahmen eingeblendet wird, und daß das zum Regeln der von der Lichtquelle abgegebenen Lichtmenge verwendete Videosignal nur innerhalb des Meßfensters (18) mit dem Integrator (19) integriert wird.

2. Einrichtung nach Anspruch 1, dadurch gekennzeichnet, daß eine manuell betätigbare Positionssteuerung (15) zum Positionieren des Meßfensters (18) innerhalb des Videobildes sowie zum Verändern der Größe des Meßfensters (18) vorgesehen ist.

3. Einrichtung nach Anspruch 1 oder 2, dadurch gekennzeichnet, daß eine Fenstereinblendeinrichtung (23) vorgesehen ist, die das Einblenden des Meßfensters (18) in das Videobild als Rahmen erlaubt.

**Europäisches Patentamt**

# EUROPÄISCHER RECHERCHENBERICHT

Nummer der Anmeldung

**EP 90 11 5366**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 257 506   (K. SIEPMANN)<br>* Spalte 4, Zeile 45 - Spalte 5, Zeile 27; Figur 1 *<br>– – – | 1 | A 61 B 1/04<br>H 04 N 5/235 |
| A | | 2,3 | |
| A | US-A-3 996 421   (M.D. PRUZNICK et al.)<br>* Zusammenfassung; Figur 1 *<br>– – – | 1-3 | |
| Y | US-A-4 834 071   (S. HOSOI et al.)<br>* Spalte 5, Zeile 28 - Spalte 6, Zeile 31; Figur 7 *<br>– – – | 1 | |
| A | EP-A-0 050 834   (OLYMPUS OPTICAL CO. LTD.)<br>* Zusammenfassung; Figur 3 *<br>– – – – – | 1 | |

| | RECHERCHIERTE SACHGEBIETE (Int. Cl.5) |
|---|---|
| | A 61 B 1/00<br>H 04 N 5/00<br>H 04 N 7/00 |

Der vorliegende Recherchenbericht wurde für alle Patentansprüche erstellt

| Recherchenort | Abschlußdatum der Recherche | Prüfer |
|---|---|---|
| Berlin | 28 November 90 | WEIHS J.A. |